# EUROPEAN PATENT APPLICATION

(11) **EP 1 736 108 A1**
(43) Date of publication of application: **27.12.2006**
(21) Application number: 06012859.2
(22) Date of filing: 22.06.2006
(51) Int. Cl.: A61B 17/34, A61B 17/00, A61B 19/00, A61B 17/22, A61M 25/00

(54) **Cathether assembly**

(30) Priority: 24.06.2005 JP 2005185733
(71) Applicant: TERUMO KABUSHIKI KAISHA, Tokyo (JP)
(72) Inventor: Okishige, Kaoru, Tsuzuki-ku Yokohama Kanagawa (JP)
(74) Representative: Casalonga, Axel

(57) **Abstract**

A catheter assembly includes a flexible tubular catheter body having a lumen open at a distal end, a negative pressure generating unit adapted to aspirate the lumen of the catheter body, and a puncture wire capable of insertion into the lumen of the catheter body. The puncture wire has at its distal end a needle with a sharp needlepoint. The needle is bent in its natural state such that the needlepoint points in a direction different from the direction of insertion of the puncture wire into the lumen.

## Description

The present invention generally relates to a catheter assembly. More specifically, the invention pertains to a catheter assembly that includes a puncture wire.

The human heart is generally divided into the right ventricle, the right atrium, the left ventricle, and the left atrium. The right atrium communicates with the superior vena cava and the inferior vena cava, and it is separated from the left atrium by the septum. The left atrium is the site where a catheter is accessed to cure cardiac arrhythmia by ablation for electrical isolation of pulmonary veins or for mitral valve annuloplasty.

Unfortunately, the left atrium presents a number of difficulties with respect to catheter access. For example, it is quite difficult, if not impossible, for a catheter to approach the left atrium through the pulmonary artery. It is also quite difficult for a catheter to approach the left atrium from the left ventricle adjoining to the left atrium through the mitral valve.

There is known a catheter or catheter assembly designed to approach the left atrium. Examples include those described in United States Patent No. 4,790,825, United States Patent No. 6,650,923 and United States Patent Application Publication No. 2004/0220461.

United States Patent No. 4,790,825 describes transseptum catheterization in which the distal opening of a catheter is intended to be inserted into the right atrium and then a needle (that passes through the septum) is projected from the distal opening for penetration into the oval fossa. In this way it is possible to form a hole in the septum and have the catheter approach the left atrium through the hole.

Unfortunately, the catheterization method described in United States Patent No. 4,790,825 suffers the disadvantage that the distal opening of the catheter is easily dislocated because it is not firmly fixed, or is simply in contact with the septum. This dislocation results in a hole at positions (such as the aorta and the right atrium) other than the septum when the needle is manipulated (or moved toward the distal end). Even though it is possible to make a hole in the septum, there is the possibility that the inner wall of the left atrium is damaged by subsequent excessive movement of the needle.

United States Patent No. 6,650,923 and United States Patent Application Publication No. 2004/0220461 describe an apparatus to detect the oval fossa by means of a sensor and bring a catheter into contact with the thus detected oval fossa.
The problems associated with the state of the art are addressed by the present invention.

According to one aspect, a catheter assembly comprises a flexible tubular catheter body possessing a distal end and a lumen which is open at the distal end of the catheter body, and a puncture wire comprising a distal end portion possessing a sharp needlepoint, with the distal end portion of the puncture wire being bent back upon itself in a natural state of the distal end portion in which a force is not applied to the distal end portion. The puncture wire is adapted to be inserted into the lumen in the catheter body with the distal end portion of the puncture wire deformed from the natural state and being adapted to be advanced along the lumen so that the sharp needlepoint moves distally beyond the distal end of the catheter and penetrates the target tissue while the distal end of the puncture wire returns toward the bent natural state following penetration.

According to another aspect, a catheter assembly comprises a flexible tubular catheter body comprising a lumen which is open at its distal end, a negative pressure generator adapted to be connected to the catheter body so as to communicate with the lumen in the catheter body to decompress the lumen of the catheter body during operation of the negative pressure generator, and a puncture wire adapted to be inserted into and advanced along the lumen of the catheter body. The puncture wire comprises a distal end portion possessing a sharp needlepoint, and the distal end portion of the puncture wire is bent in its natural state such that the needlepoint points in a direction different from a direction of advancement of the puncture wire.

A method of setting up a catheter at a living tissue comprises inserting a catheter into a blood vessel, with the catheter comprising a lumen which is open at a distal end of the catheter, bringing the distal end of the catheter close to a target tissue, inserting a puncture wire into the lumen of the catheter, wherein the puncture wire comprises a distal end portion that is bent in a natural state of the puncture wire, and wherein the distal end portion possesses a sharp needlepoint. The method also involves advancing the puncture wire through the lumen of the catheter to cause the sharp needlepoint of the puncture wire to move out the distal end of the catheter and penetrate the target tissue with the sharp needlepoint of the puncture wire, with the distal end portion of the puncture wire bending toward its natural state following penetration of the target tissue by the sharp needlepoint.

When used to cure cardiac arrhythmia, the catheter assembly is transfixed into the oval fossa (living tissue) through the septum of the heart (right atrium) such that its needle is curved or bent in its natural state and the needlepoint points in a direction different from that in which the distal end port points. This generally inhibits or prevents the needlepoint from pointing to any parts other than the septum (e.g., the wall (living tissue) of the left atrium behind the septum). Thus the needle will not inadvertently transfix the above-mentioned part but will reliably and safely transfix the septum.

Moreover, the catheter body has a distal end opening which functions as a suction port to adhere to the living tissue. This function prevents the distal opening from being dislocated relative to the living tissue. In other words, the distal opening is reliably fixed to the living tissue. In this way, the catheter reliably transfixes the living tissue at the target site.

Fig. 1 is a side view of the entire catheter assembly disclosed herein.

Fig. 2 is an enlarged view of the region A of the catheter assembly shown in Fig. 1.

Fig. 3 is a partial longitudinal sectional view of the hub of the catheter assembly shown in Fig. 1.

Fig. 4 is a perspective view of the valve installed in the hub shown in Fig. 3.

Fig. 5 is an operational diagram illustrating one example of a manner of using the catheter assembly shown in Fig. 1.

Fig. 6 is an operational diagram illustrating another example of a manner of using the catheter assembly shown in Fig. 1.

Fig. 7 is an operational diagram illustrating another example of a manner of using the catheter assembly shown in Fig. 1.

Fig. 8 is an operational diagram illustrating a further example of a manner of using the catheter assembly shown in Fig. 1.

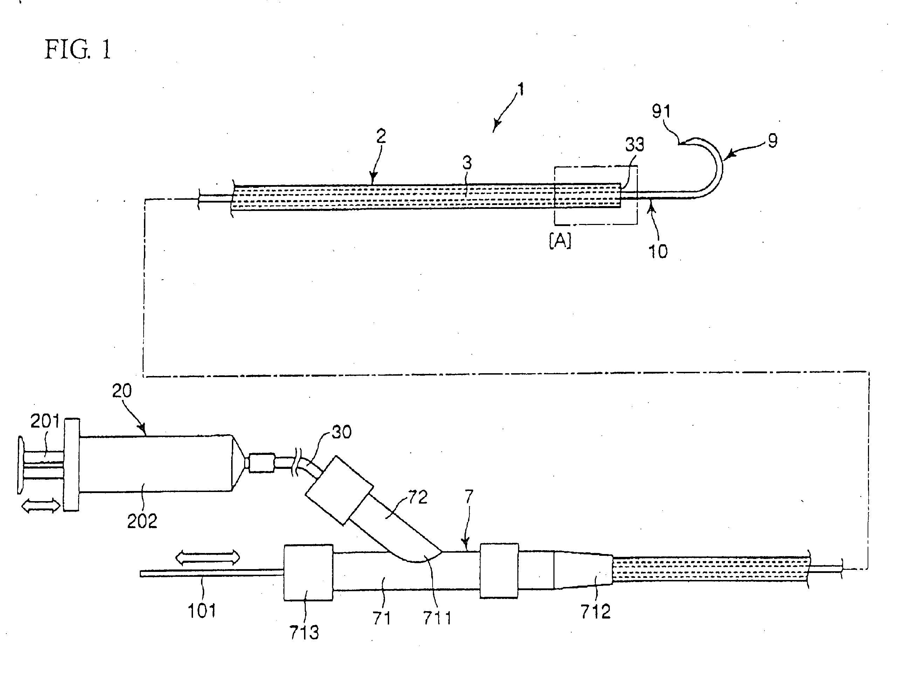

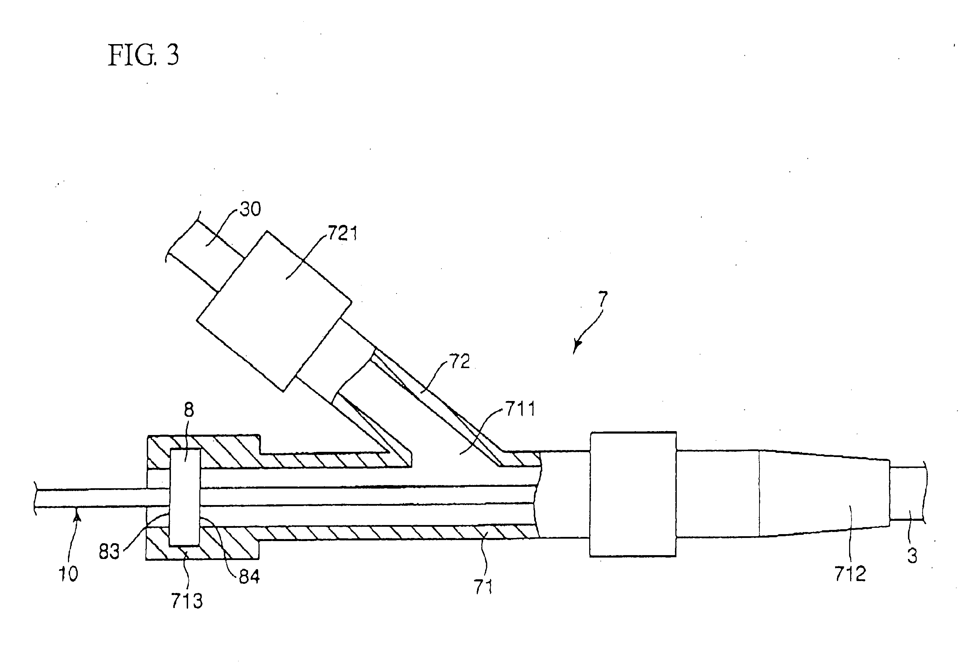

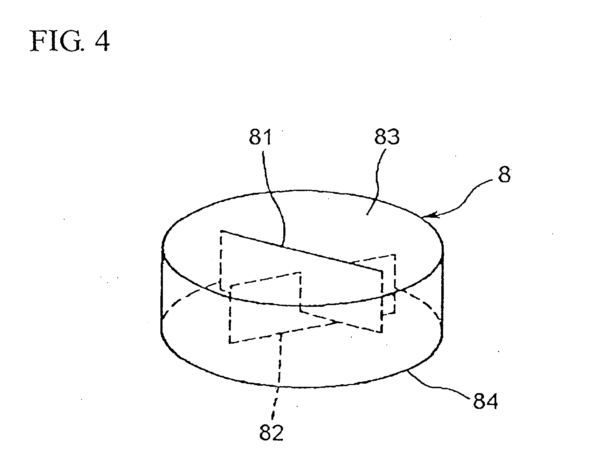

In Figs. 1 and 3, the right side is referred to as the "distal end" and the left side is referred to as the "proximal end". In Fig. 4, the upper side is referred to as the "proximal end" and the lower side is referred to as the "distal end".

As shown in Fig. 1, the catheter assembly 1 is comprised of a catheter 2, a puncture wire 10, and a syringe 20 which operates as a negative pressure generator or suction means. The catheter 2 includes a flexible tubular catheter body 3 and a Y-shaped branching connector 7 detachably mounted on a proximal hub 712 of the catheter body 3.

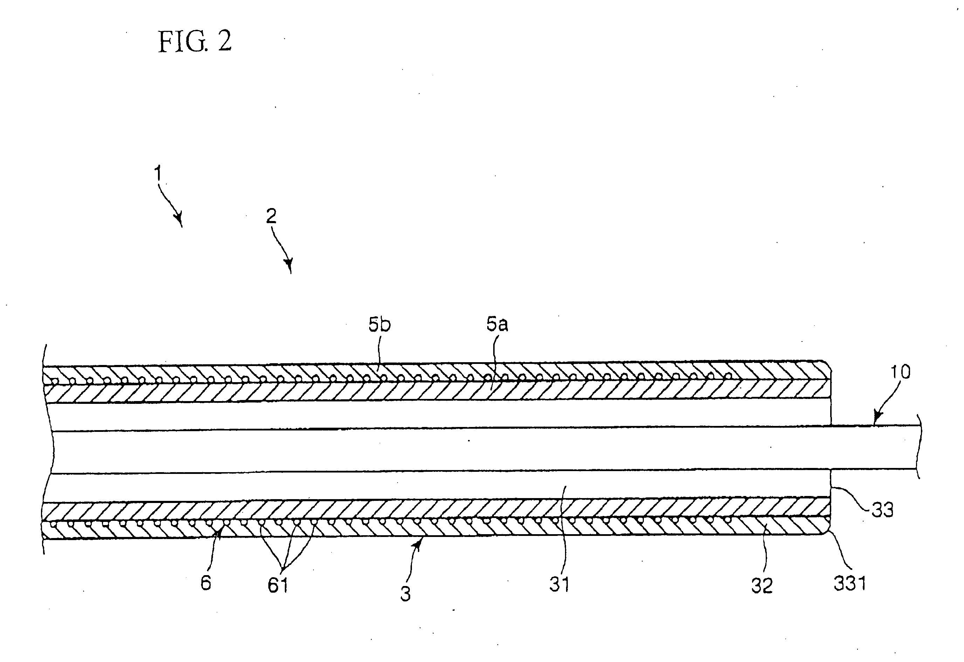

As shown in Fig. 2, the catheter body 3 has a lumen 31 extending over almost the entire length of the catheter body. This lumen 31 has an opening at its distal end. The lumen 31 functions as a passage for the transfer of liquid, such as contrast medium, drug solution, and/or cleaning solution, and also as a guide for a treatment catheter.

The distal opening 33 of the catheter body 3 permits liquid to be ejected into the lumen 31 and/or permits the catheter being guided to project from it.

In addition, the distal opening 33 of the catheter body 3 has a round edge 331 to facilitate smooth insertion of the catheter 2 into a living body (blood vessel) while also protecting the vessel wall from being damaged. Thus, the rounded edge contributes to operability and safety.

The catheter body 3 should preferably be of laminate structure comprised of more than one layer. That is, as shown in Fig. 2, the catheter body 3 is comprised of an inner layer 5a and an outer layer 5b.

The inner and outer layers 5a and 5b may be formed from one or more materials (in combination) selected from thermoplastic resins and elastomers, such as polyolefin including polyethylene, polypropylene, ethylene-propylene copolymer, ethylene-vinyl acetate copolymer, and crosslinked ethylene-vinyl acetate copolymer, polyvinyl chloride, polyester (PET, PBT, PEN, etc.), polyamide, polyimide, polyurethane, polystyrene, polycarbonate, fluoroplastics (poly-tetrafluoroethylene etc.), silicone resin, silicone rubber, and other elastomers (including polyamide and polyester thermoplastic elastomers). The inner and outer layers 5a and 5b may be formed from the same materials or different materials.

In the case where the inner and outer layers 5a and 5b are formed from different materials, the materials may differ in flexibility/rigidity.

The inner layer 5a may be formed from a highly slippery material, such as poly-tetrafluoroethylene, fluorinated ethylene-propylene copolymer (FEP), and high-density polyethylene. The outer layer 5b may be formed from comparatively soft plastics, such as polyurethane, polyamide, and polyester. These materials permit the puncture wire 10 and the guide wire to move (slide) smoothly in the lumen 31 of the catheter body 3. Moreover, they also permit the catheter body 3 to smoothly slide at the time of insertion into the blood vessel, thus contributing to smooth and reliable insertion.

As shown in Fig. 2, it is desirable that a reinforcing member 6 is positioned (embedded) in the wall of the catheter body 3, for example between the inner layer 5a and the outer layer 5b. By virtue of the reinforcing member 6, the catheter 2 and the catheter assembly 1 exhibit good torque transmitting performance, pushability, kinking resistance, and followability. This leads to improved operability at the time of insertion into the blood vessel and pressure resistance for the internal pressure (liquid pressure) in the lumen 31.

The reinforcing member 6 is arranged over almost the entire length of the catheter body 3. As shown in Fig. 2, the reinforcing member 6 is not arranged in the distal end 32 of the catheter body 3. This structure helps impart flexibility to the distal end and contributes to the safe and reliable insertion into the blood vessel.

The reinforcing member 6 may be formed from a filamentous body 61. The filamentous body 61 is preferably in the form of braid or coil. The filamentous body 61 is given a desired strength according to the material, diameter, mesh size and number of coil windings which cab be appropriately selected.

The filamentous body 61 may be made of metallic wire (such as stainless steel, tungsten, piano wire, and Ni-Ti alloy), plastic fiber (such as aramid and Kevlar), and carbon fiber.

The diameter (outer diameter) of the filamentous body 61 is not specifically restricted, though it should preferably be about 3 to 70_m, more preferably about 20 to 40 _m.

The catheter body 3 comprised of the inner layer 5a, the outer layer 5b and the reinforcing member 6 interposed between the inner and outer layers may be produced by placing the reinforcing member 6 on the surface of the inner layer 5a, covering the reinforcing member 6 with the outer layer 5b, and integrating them by heating. A heat-shrinkable tube may be used as the outer layer 5b. Alternatively, either of the inner layer 5a and the outer layer 5b may be a coating film formed by coating, dipping, or spraying.

The catheter body 3 may also be of a single-layer structure. In the case of such a structure, the reinforcing member 6 should preferably be embedded in the layer constituting the catheter body 3.

Although not shown, the distal end 32 (or any other part) of the catheter body 3 may have a ring-shaped or coil-shaped member embedded therein which possesses radiopaque characteristics, especially x-ray opaque characteristics. Such a member helps facilitate confirmation of the position of the distal end 32 of the catheter body 3 in the living body under radioscopy. The distal end 32 may be made radiopaque by incorporating the resin constituting the inner layer 5a or the outer layer 5b with 30 to 70 wt% of radiopaque metal powder, such as barium sulfate, bismuth oxide, and/or tungsten.

The Y-shaped branching connector 7 is provided at the proximal end of the catheter 3 and is detachably mounted on the catheter body 3. As shown in Figs. 1 and 3, the Y-shaped branching connector 7 is comprised of the connector body 71 and the branch part 72 which branches off in the neighborhood of the center 711 of the connector body 71.

The connector body 71 has a tubular shape. The connector 7 is detachably connected to the hub 712 that is fixed the proximal end of the catheter body 3, so that the lumen 31 of the catheter body 3 communicates with an inner space in the connector body 71.

As illustrated in Fig. 3, a valve 8 is installed at the proximal end 713 of the connector body 71. In use, the puncture wire 10 or the guide wire is inserted into the catheter body 3, specifically the lumen 31, through the valve 8.

The branch part 72 has a tubular shape, and communicates with the connector body 71 in the neighborhood of the center portion 711 of the body 71. The branch part 72 projects or extends in a direction aslant with respect to the central axis of the connector body 71 so that the branch part 72 and the connector body 71 form an angle relative to one another.

The syringe 20 is adapted to be connected to the end 721 of the branch part 72 through the tube 30 as shown in Fig. 1.

It should thus be noted that the Y-shaped branching connector 7 comprises two parts, one for insertion and withdrawal of the puncture wire 10 and the guide wire, and the other for connection of the syringe 20. This construction prevents the operation for insertion and withdrawal of the puncture wire 10 and the guide wire into and from the catheter body 3 from producing an adverse effect on the operation for negative pressure generation (the operation of the syringe 20) for the catheter body 3 (the lumen 31). The result is an improvement in the operability of the catheter assembly 1. The adverse effect produced by insertion and withdrawal of the puncture wire 10 and the guide wire can create difficulties in maintaining the negative pressure in the lumen 31.

The Y-shaped branching connector 7 may be formed from any material and so the particular material is not specifically restricted. Various metallic materials and plastic materials may be used alone or in combination with one another. A preferred material is plastic having high transparency such as polycarbonate.

As shown in Fig. 1, the syringe 20 is comprised of an outer cylinder 202 and a plunger 201. A gasket is fixed to the distal end of the plunger 201 and slides within the outer cylinder 202.

The syringe 20 aspirates fluid (such as saline previously injected into the catheter 2) from the lumen 31 of the catheter body 3 as the plunger 201 is pulled out from the outer cylinder 202. If this operation (for suction) is carried out while the distal opening 33 of the catheter body 3 is in close contact with the living tissue (such as the septum of the heart), the inside of the lumen 31 is decompressed in a reliable manner. The syringe 20 preferably functions to fix the position of the plunger 201 in the pulled state.

As mentioned above, the valve 8 is installed at the proximal end 713 of the connector body 71. The valve 8 is shown in Fig. 4 and is in the form of an elastic flat disc.

The valve 8 has a first slit 81 and a second slit 82, which are opened and closed as the puncture wire 10 and the guide wire are inserted and withdrawn. As shown in Fig. 4, the first slit 81 is formed such that it reaches only the proximal end (top) of the valve 8 from the inside of the valve 8. The first slit 81 is straight in its plan view so that it possesses a relatively simple shape or configuration. Because of its relatively simple shape, the first slit 81 is reliably and easily opened and closed.

In addition, the second slit 82 is formed such that it reaches only the distal end or bottom end of the valve 8 from the inside of the valve 8. The second slit 82 is also straight in its plan view so that it too possesses a relatively simple shape or configuration. Because of its relatively simple shape, the second slit 82 is reliably and easily opened and closed.

The first and second slits 81, 82 partially cross each other in the valve 8 as illustrated in Fig. 4. The two slits 81, 82 intersect each other at right angles, although intersection at any angle is permissible.

The valve 8 constructed as mentioned above remains closed regardless of whether the puncture wire 10 passes through the valve 8 or the puncture wire 10 has been withdrawn from the valve 8. Thus, the valve 8 maintains the negative pressure in the lumen 31 when the syringe 20 is operated, with the distal opening 33 of the catheter 2 in close contact with the living tissue.

The valve 8 may be formed from any material, such as natural rubber, synthetic rubber (including isoprene rubber, silicone rubber, urethane rubber, styrene-butadiene rubber, fluororubber, and acrylic rubber), and thermoplastic elastomers (including polyamides and polyesters).

The structure of the valve 8 is not limited to that mentioned above. For example, the valve 8 may be a ring-shaped valve constructed such that its inside diameter shrinks as it is tightened by rotating the cap-like proximal end 713 of the connector body 71.

As shown in Fig. 1, the puncture wire 10 is a flexible wire body adapted to be inserted into the lumen 31 of the catheter body 3. The distal end portion of the puncture wire 10 comprises a needle 9.

The needle 9 possesses a sharp needlepoint 91. The needle 9 is bent (e.g., curved) in its natural state so that the needlepoint 91 points in a direction different from the forward axial direction of the puncture wire 10. That is, the needle 9 is bent back upon itself and configured so that it does not continue along the longitudinal extent of the portion of the wire 10 adjoining the needle 9 and is not colinear with the portion of the wire 10 adjoining the needle 9. The needlepoint 91 preferably turns approximately toward the proximal end so that the needle is generally bent back upon the portion of the wire 10 adjoining the needle 9. In other words, the needle 9 roughly takes on a J shape in its natural state. The term "natural state" means that no external force is applied to the needle.

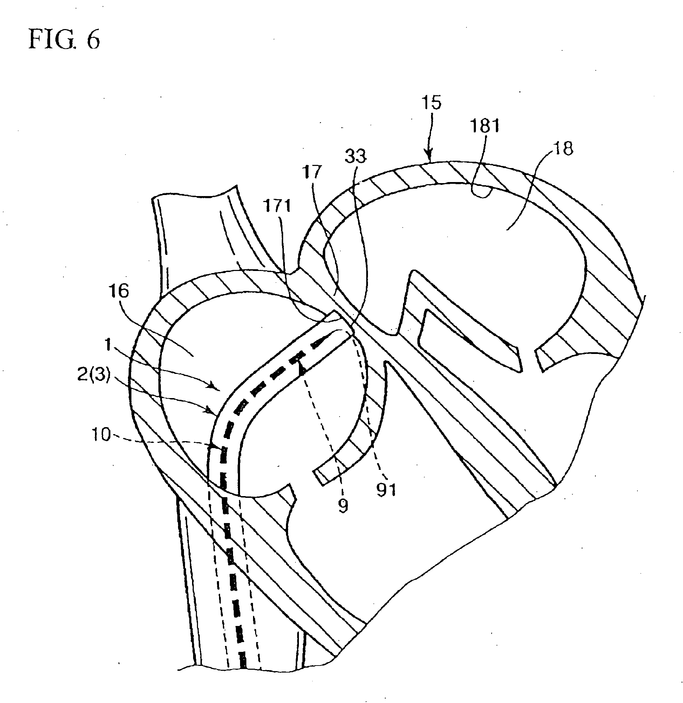

When the puncture wire 10 is inserted from the valve 8 of the Y-shaped branch connector 7 such that the needle 9 is held in the lumen 31, the needle 9 takes on a stretched shape as shown in Fig. 6 because it is constrained by the inside of the lumen 31. In other words, the puncture wire 10 is less rigid (more flexible) than the catheter body 3.

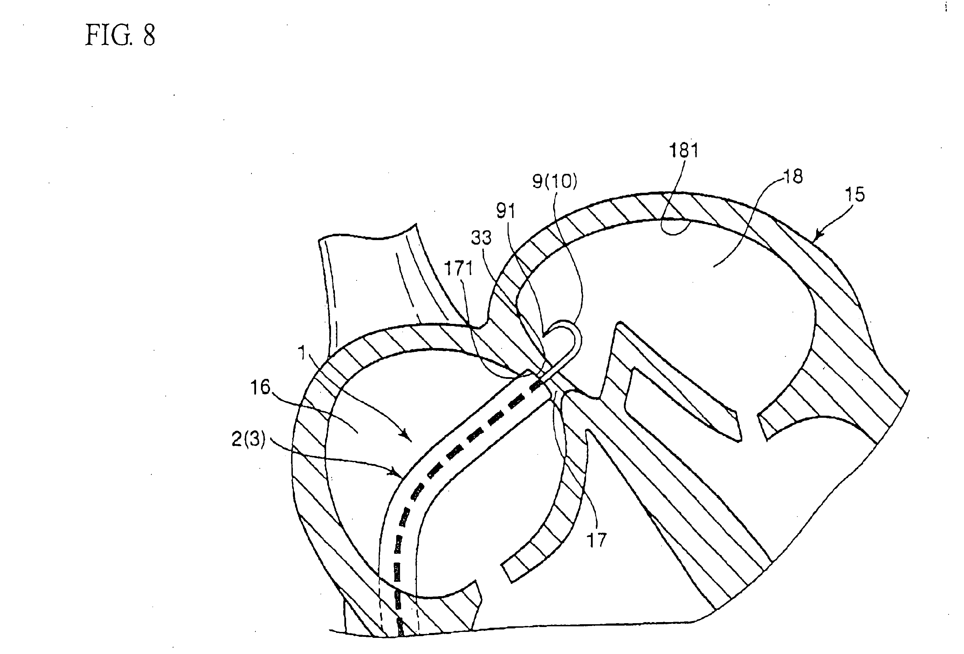

The needle 9 in its stretched state returns to its J-shape after the needlepoint 91 punctures the living tissue at the target site as shown in Fig. 8. Thus the needlepoint 91 never sticks in (or damages) the other living tissue which exists at the position to which it projects from the opening 33. This leads to safe and reliable penetration into the living tissue.

As shown in Fig. 1, the outside diameter of the puncture wire 10 over its entire length is smaller than the inside diameter of the catheter body 3. This helps facilitate an easy and reliable insertion of the puncture wire 10 into the lumen 31.

The puncture wire 10 has an outside diameter of preferably about 0.25 to 1.5 mm, more preferably about 0.5 to 1.0 mm, though such outside diameter depends on the inside diameter of the catheter body 3.

The puncture wire 10 (filamentous body) may be formed from any metallic material, such as stainless steel, cobalt alloy, pseudoelastic alloy (including superelastic alloy), and piano wire.

Examples of stainless steel include SUS304, SUS303, SUS316, SUS316L, SUS316J1, SUS316J1L, SUS405, SUS430, SUS434, SUS444, SUS429, SUS430F, SUS302, etc.

With the puncture wire 10 made of cobalt alloy, the wire is less liable to buckling, owing to its relatively high modulus of elasticity and adequate elastic limit. The cobalt alloy is not specifically restricted so long as it contains cobalt as a major constituent element. Preferably, an alloy which contains mainly Co in the weight ratio is used. More preferably, a Co-Ni-Cr alloy is used for the puncture wire 10 to produce the above-mentioned effect. This alloy has a relatively high elastic modulus and a high elastic limit suitable for cold molding. Therefore, it can be made into a thin wire with relatively high buckling resistance, and adequate flexibility and rigidity for insertion to the target site.

A preferred example of the Co-Ni-Cr alloy is one which is composed of 28 to 50 wt% of Co, 10 to 30 wt% ofNi, and 10 to 30 wt% of Cr, with the remainder being Fe. The constituent element may be partially replaced with any other element (substituent element) so as to obtain the various characteristics associated with each element. For example, the strength of the puncture wire 10 can be improved by selecting at least one substituent element such as Ti, Nb, Ta, Be, or Mo. The content of any elements (excluding Fe) other than Co, Ni, and Cr should preferably be less than 30 wt%.

It is possible to replace Co, Ni, or Cr partly with any other element. For example, part of the Ni may be replaced with Mn for improved processability. Part of the Cr may be replaced with Mo and/or W for improved elastic limit. Among particularly desirable Co-Ni-Cr alloys is Co-Ni-Cr-Mo alloy (containing Mo).

Typical examples of the Co-Ni-Cr alloys are listed below.
(1) 40 wt% Co - 22 wt% Ni - 25 wt% Cr - 2 wt% Mn - 0.17 wt% C - 0.03 wt% Be - remainder Fe
(2) 40 wt% Co - 15 wt% Ni - 20 wt% Cr - 2 wt% Mn - 7 wt% Mo - 0.15 wt% C - 0.03 wt% Be - remainder Fe
(3) 42 wt% Co - 13 wt% Ni - 20 wt% Cr - 1.6 wt% Mn - 2 wt% Mo - 2.8 wt% W - 0.2 wt% C - 0.04 wt% Be - remainder Fe
(4) 45 wt% Co - 21 wt% Ni - 18 wt% Cr - 1 wt% Mn - 4 wt% Mo - 1 wt% Ti - 0.02 wt% C - 0.3 wt% Be - remainder Fe
(5) 34 wt% Co - 21 wt% Ni - 14 wt% Cr - 0.5 wt% Mn - 6 wt% Mo - 2.5 wt% Nb - 0.5 wt% Ta - remainder Fe
The term "Co-Ni-Cr" alloy embraces these alloys.

The pseudoelastic alloy (which exhibits pseudoelasticity) is comparatively flexible and easily restores its original shape, thus resisting being permanently curved. When the puncture wire 10 is made of this material, its distal end (needle 9) has sufficient flexibility and ability to restore its original shape. Thus, because of its ability to restore its original shape even after repeated bending and flexing, the puncture wire 10 does not become permanently curved and hence retains is good operability.

The pseudoelastic alloys include those which give any stress-strain curve due to tensile force and also include those which possess or lack apparent transformation points such as As, Af, Ms, and Mf. In other words, they include any alloy which greatly deforms under stress and restores its original shape almost completely upon removal of stress.

The pseudoelastic alloy includes superelastic alloys. Preferred examples of superelastic alloys include Ni-Ti alloy containing 49 to 52 at% Ni, Cu-Zn alloy containing 38.5 to 41.5 wt% Zn, Cu-Zn-X alloy containing 1 to 10 wt% (X = at least one species of Be, Si, Sn, Al, and Ga), and Ni-Al alloy containing 36 to 38 at% Al. Of these examples, Ni-Ti alloy is most desirable.

The needle 9 of the puncture wire 10 should preferably be covered with a surface coating of low-friction material so that the puncture wire 10 can be smoothly inserted into and withdrawn from the lumen 31. In this way, the operability is enhanced. Moreover, the needle 9 with surface coating smoothly punctures the living tissue, thereby reducing the patient's load. The low-friction material should preferably be a hydrophilic material which exhibits lubricating property in its moistened state. The surface coating of the low-friction material may be made both on the outside of the puncture wire 10 and on the inside of the catheter body 3 or only on the inside of the catheter body 3.

The needle 9 may take on any shape other than the J-shape (e.g., a L-shape). The shape should preferably be such that the distal end of the needle is bent more than 90° with respect to the lengthwise direction of the remaining portion of the needle.

The puncture wire 10 should preferably be longer (in overall length) than the catheter 2 so that it can be easily manipulated by holding its proximal end 101.

The puncture wire 10 should preferably be more flexible than the catheter body 3 so that the needle 9 readily takes on a generally straight configuration in the lumen 31.

The catheter assembly 1 of the present invention takes on one of two states when the catheter 2 and the puncture wire 10 are assembled. In one state, the needle 9 is retracted into the lumen, and in the other state the needle 9 projects from the distal opening 33. The two states are hereinafter described as the "retracted state" and the "projecting state" respectively.

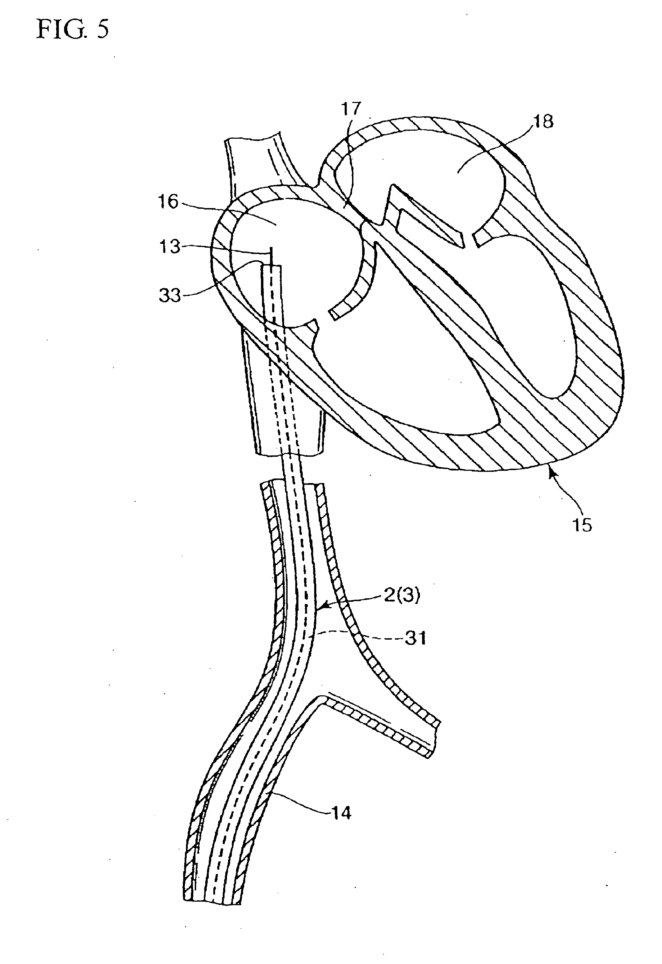

One example of use of the catheter assembly 1 described above is explained below with reference to Figs. 5-8. All operations are carried out by observing the position and posture of the catheter assembly 1, for example under X-ray radiography.

[1] First, an introducer sheath (not shown) is used to insert into the femoral vein 14 along a short guide wire (not shown) arranged beforehand at the vein 14. The catheter 2 is inserted into the sheath, with the guide wire 13 previously inserted into the lumen 31 of the catheter body 3. The distal opening 33 of the catheter 2 is inserted into the femoral vein 14 through the distal opening of the sheath, with the guide wire 13 preceding.

Then, as shown in Fig. 5, the distal opening 33 of the catheter 2 is inserted into the right atrium 16 of the heart 15, with the guide wire 13 slowly advancing toward the heart 15.

[2] The guide wire 13 is then withdrawn from the catheter 2. Then, as shown in Fig. 6, the puncture wire 10 is inserted into the catheter 2. At this time, the catheter assembly 1 is in the retracted state. The distal end portion of the puncture wire is stretched or deformed from its bent natural state (i.e., is somewhat straightened) when positioned in the lumen of the catheter body. The puncture wire 10 is moved or advanced such that the needle 9 (or the needle point 91) is slightly retracted in the proximal direction (toward the Y-shaped branching connector 7) from the distal opening 33 of the catheter 2.

As shown in Fig. 6, the distal opening 33 or distal end of the catheter 2 is brought into close contact with the oval fossa 171 of the septum 17 (hereinafter this state is referred to as the "contact state") by observation under X-ray radiography. While the contact state is maintained, the syringe 20 connected to the Y-shaped branching connector 7 of the catheter 2 is operated to effect a sucking action (aspirating). In this way, fluid is withdrawn from the lumen 31 so that the lumen 31 is decompressed or somewhat collapsed, and the distal opening 33 of the catheter sticks or if fixed to the oval fossa 171. The distal opening 33 sticking to the oval fossa 171 thus becomes firmly fixed to the oval fossa 171 without the possibility of displacement.

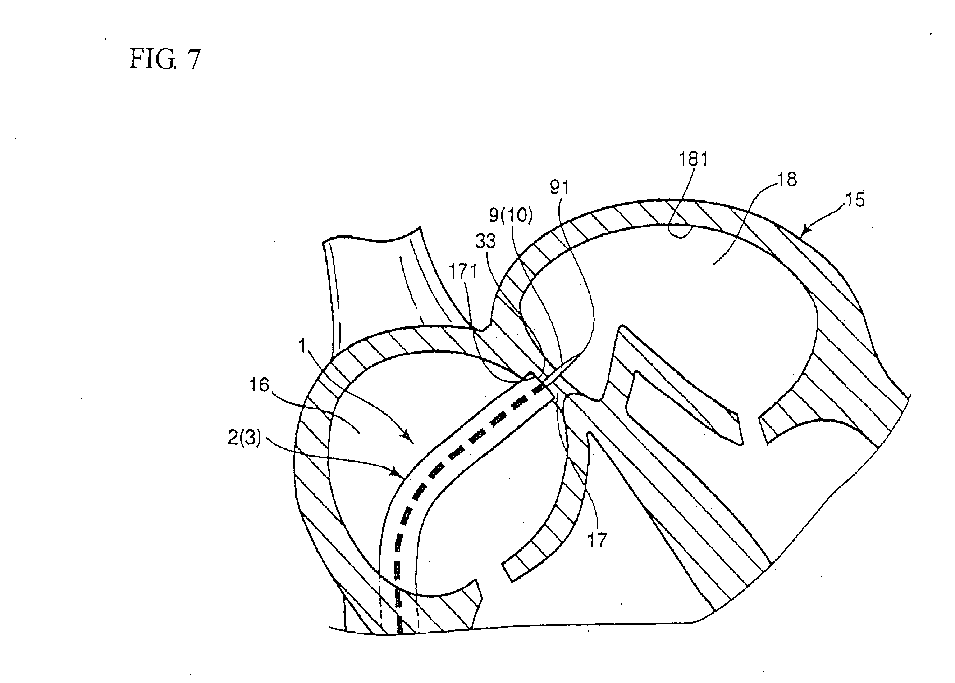

[3] While the catheter assembly is still in the contact state or decompressed state, the puncture wire 10 is pushed or advanced distally within the catheter body 3 as shown in Fig. 7 so that the needle point 91 passes through the distal opening 33 and punctures the oval fossa 171. The needle 91 is able to reliably puncture the oval fossa 171 because the distal end 33 of the catheter body 3 is reliably fixed to the oval fossa 171 as mentioned above.

After the needlepoint 91 has punctured the oval fossa 171, the catheter assembly 1 assumes the projecting state and the needle 9 takes on its natural state (e.g., J shape) in the left atrium 18 as shown in Fig. 8.

The bent needle 9 (with the needlepoint 91) is thus prevented from puncturing or otherwise damaging the wall 181 of the left atrium 18, even though the puncture wire 10 is pushed further into the left atrium 18 to such an extent that the needle 9 reaches the wall 181 of the left atrium 18.

[4] The catheter 2 is advanced along the puncture wire 10 so that the distal end 32 of the catheter 2 enters the left atrium 18. Then, the puncture wire 10 is withdrawn from the catheter 2, with the distal end 32 of the catheter 2 remaining positioned in the left atrium 18. The thus placed catheter 2 can be used for insertion of any device for treatment or diagnosis into the left atrium 18.

An example of a device for treatment includes an ultrasonic ablation catheter, although other devices for treatment can be used.

An example of a device for diagnosis includes a sensor-carrying catheter, although once again other devices for diagnosis are possible.

In the embodiment of the catheter assembly described above, the negative pressure generator is a syringe. However, other negative pressure generators can also be employed such as a suction pump.

The principles, preferred embodiment and mode of operation of the disclosed catheter assembly have been described in the foregoing specification. However, the invention which is intended to be protected is not to be construed as limited to the particular embodiment disclosed. Further, the embodiment described herein is to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

## Claims

1. A catheter assembly comprising:
a flexible tubular catheter body possessing a distal end, the catheter body comprising a lumen which is open at the distal end of the catheter body;
a puncture wire comprising a distal end portion possessing a sharp needlepoint;
the distal end portion of the puncture wire being bent back upon itself in a natural state of the distal end portion in which a force is not applied to the distal end portion; and
the puncture wire being adapted to be inserted into the lumen in the catheter body with the distal end portion of the puncture wire deformed from the natural state and being adapted to be advanced along the lumen so that the sharp needlepoint moves distally beyond the distal end of the catheter and penetrates the target tissue while the distal end of the puncture wire returns toward the bent natural state following penetration.

2. The catheter assembly as set forth in Claim 1, further comprising a connector positioned at a proximal end of the catheter body, the connector comprising a tubular body and a branch that branches off from the tubular body at a middle portion of the tubular body, the tubular body comprising a valve that is adapted to selectively open and close.

3. The catheter assembly as set forth in Claim 1, further comprising a negative pressure generator adapted to be communicated with the lumen in the catheter body to withdraw fluid from the lumen of the catheter body during operation of the negative pressure generator.

4. A catheter assembly comprising:
a flexible tubular catheter body, the catheter body comprising a lumen which is open at its distal end;
a negative pressure generator adapted to be connected to the catheter body so as to communicate with the lumen in the catheter body to decompress the lumen of the catheter body during operation of the negative pressure generator;
a puncture wire adapted to be inserted into and advanced along the lumen of the catheter body, the puncture wire comprising a distal end portion possessing a sharp needlepoint;
the distal end portion of the puncture wire being bent in its natural state such that said needlepoint points in a direction different from a direction of advancement of the puncture wire.

5. The catheter assembly as set forth in Claim 4, wherein the opening is adapted to be in close contact with a living tissue while the lumen is decompressed by the negative pressure generator.

6. The catheter assembly as set forth in Claim 5, wherein the needlepoint punctures the living tissue through the opening as the puncture wire is moved toward the distal end of the catheter body while the opening is in close contact with the living tissue.

7. The catheter assembly as set forth in Claim 4, wherein the distal end of the puncture wire is substantially J-shaped in its natural state.

8. The catheter assembly as set forth in Claim 4, wherein the distal end of the puncture wire is stretched while it is accommodated in the lumen of the catheter body.

9. The catheter assembly as set forth in Claim 4, wherein the puncture wire along its entire length has an outer diameter smaller than an inner diameter of the lumen.

10. The catheter assembly as set forth in Claim 4, wherein the puncture wire is made of superelastic alloy.

11. The catheter assembly as set forth in Claim 4, wherein the negative pressure generator is a syringe that aspirates fluid in the lumen of the catheter body.

12. The catheter assembly as set forth in Claim 4, wherein the catheter body comprises a hub at a proximal end of the catheter body.

13. The catheter assembly as set forth in Claim 12, further comprising a connector detachably mounted to the hub and comprising a tubular body and a branch that branches off from the tubular body in a middle portion of the tubular body.

14. The catheter assembly as set forth in Claim 13, wherein the tubular body comprises a valve that is adapted to selectively open and close.

15. The catheter assembly as set forth in Claim 14, wherein the puncture wire is adapted to be inserted into the catheter body through the valve.

16. The catheter assembly as set forth in Claim 15, wherein the valve remains closed regardless of whether the puncture wire is present or absent.

17. The catheter assembly as set forth in Claim 13, wherein the negative pressure generator is connected to the branch.
